# DEMANDE DE BREVET EUROPEEN

(11) **EP 0 989 111 A1**
(43) Date de publication de la demande: **29.03.2000**
(21) Numéro de dépôt: 99402296.0
(22) Date de dépôt: 20.09.1999
(51) Int. Cl.: C07C 69/732, C07C 69/734, A61K 7/48, C07C 59/42, C07C 59/58

(54) **Nouveaux dérivés de l'acide 10-hydroxy-2-décénoique et utilisation dans une composition destinée à favoriser la desquamation de la peau, et composition le comprenant**

(30) Priorité: 22.09.1998 FR 9811811
(71) Demandeur: L'OREAL, 75008 Paris (FR)
(72) Inventeur: Maignan, Jean, 93290 Tremblay-en-France (FR); Genard, Sylvie, 75012 Paris (FR)
(74) Mandataire: Tezier Herman, Béatrice

(57) **Abrégé**

L'invention concerne de nouveaux dérivés de l'acide 10-hydroxy-2-décénoique, l'utilisation en tant que composé actif dans une composition d'une quantité efficace d'acide 10-hydroxy-2-décénoique ou d'au moins l'un de ses dérivés, l'acide ou la composition étant destinés à favoriser la desquamation de la peau et/ou stimuler le renouvellement épidermique et/ou lutter contre le vieillissement de la peau. Elle se rapporte aussi à des compositions pour favoriser la desquamation de la peau et/ou stimuler le renouvellement épidermique et donc lutter contre le vieillissement cutané intrinsèque et/ou extrinsèque, ainsi qu'à un procédé de traitement non thérapeutique de la peau destiné à favoriser la desquamation et/ou lutter contre le vieillissement de la peau.

## Description

L'invention concerne de nouveaux dérivés de l'acide 10-hydroxy-2-décénoique l'utilisation en tant que composé actif dans une composition, d'une quantité efficace d'acide 10-hydroxy-2-décénoique ou d'au moins l'un de ses dérivés, l'acide ou la composition étant destinés à favoriser la desquamation de la peau et/ou stimuler le renouvellement épidermique et/ou lutter contre le vieillissement de la peau. Elle se rapporte aussi à des compositions pour favoriser la desquamation de la peau et/ou stimuler le renouvellement épidermique et donc lutter contre le vieillissement cutané intrinsèque et/ou extrinsèque, ainsi qu'à un procédé de traitement non thérapeutique de la peau destiné à favoriser la desquamation et/ou lutter contre le vieillissement de la peau.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement.
Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée *stratum corneum.*

Le vieillissement cutané résultant de facteurs intrinsèques ou extrinsèques se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté ou par l'apparition de télangiectasies.

Certains de ces signes du vieillissement sont plus particulièrement liés au vieillissement intrinsèque ou physiologique, c'est-à-dire au vieillissement « normal » lié à l'âge ou chronobiologique, alors que d'autres sont plus spécifiques du vieillissement extrinsèque, c'est-à-dire du vieillissement provoqué d'une manière générale par l'environnement ; il s'agit plus particulièrement du photovieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.
Par ailleurs, de nombreux brevets et publications (voir par exemple la demande EP-A-413528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les β-hydroxyacides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau en favorisant la desquamation, c'est-à-dire l'élimination des cellules « mortes » situées à la surface de la couche cornée de l'épiderme. Cette propriété "desquamante" est aussi appelée, souvent à tort, propriété kératolytique.

Mais les composés de l'art antérieur présentent également des effets secondaires, qui consistent en des picotements, des tiraillements, des échauffements et des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

L'invention a pour but de pallier les inconvénients des solutions de l'art antérieur et de proposer de nouveaux composés favorisant la desquamation de la peau et/ou stimulant le renouvellement épidermique, dont l'utilisation n'entraînerait pas de picotements, de tiraillements, d'échauffements ou de rougeurs désagréables pour l'utilisateur.

L'acide 10-hydroxy-2-décénoique est présent sous forme Trans dans la gelée royale dont il constitue environ 10% de la masse sèche, et dont il a été extrait pour la première fois en 1940 par Townsend et Lucas (Biochem. J., 34, 1155 (1940)).

L'acide 10-hydroxy-2-décénoique sous forme Trans intervient dans la différenciation des castes femelles chez les abeilles.

Dans l'art antérieur, cet acide est connu pour être utilisé dans des compositions pour la prévention des leucémies, des compositions dépigmentantes, des composition ayant une activité sur la chute et la repousse des poils et des cheveux, des compositions anti-séborrhéiques, des compositions anti-tumorales ou encore bactériostatiques.

A la connaissance de la demanderesse il n'a jamais été décrit dans l'état de la technique l'utilisation de l'acide 10-hydroxy-2-décénoique ou d'au moins un de ses dérivés pour favoriser la desquamation de la peau et/ou stimuler le renouvellement épidermique et donc lutter contre le vieillissement cutané intrinsèque et/ou extrinsèque.

La demanderesse a trouvé de manière inattendue que l'application d'une quantité efficace d'acide 10-hydroxy-2-décénoique ou d'au moins un de ses dérivés permet de favoriser la desquamation de la peau et/ou stimuler le renouvellement épidermique donc de lutter contre le vieillissement.

Ainsi, l'invention a pour objet l'utilisation dans une composition d'au moins un composé répondant à la formule générale (I): 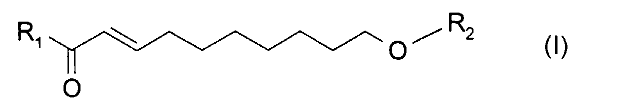 dans laquelle
R₁ représente un radical ou un atome choisi parmi :
   - un atome d'hydrogène
   - un radical -NR'R" dans lequel R' et R" identiques ou différents représentent un atome d'hydrogène ou un radical alkyle en C₁-C₄ ou un radical alcényle en C₃-C₄ ou un radical cyclopentyle ou un radical cyclohexyle, R' et R" pouvant former un hétérocycle avec l'atome d'azote auquel ils se rattachent, le radical -NR'R" pouvant, en outre, être le reste d'un acide aminé ou de la glucosamine.
   - un radical -OR₃, dans lequel R₃ représente un radical ou un atome choisi parmi :
      - un atome d'hydrogène ;
      - un radical alkyle linéaire ou ramifiée en C₁ à C₁₈ ;
      - un radical alcényle linéaire ou ramifiée en C₂ à C₁₈ ou
      - un radical perfluoroalkyle linéaire ou ramifiée en C₁ à C₁₈ ;
      les radicaux alkyle ou alcényle pouvant être substituées par un ou plusieurs groupements hydroxyles et/ou amino et/ou aminoalkyles ; ces radicaux peuvent en outre être substitués par une fonction acide carboxylique ou une fonction carboxylate d'alkyle linéaire ou ramifié en C₁-C₁₈ ;
   - un radical aryle ou un radical aralkyle éventuellement substitués par des radicaux alkyles en C₁-C₁₈ linéaires ou ramifiés, des radicaux acyles -CO-R"', des radicaux alcoxy -OR"', dans lesquels R"' représente un atome d'hydrogène ou un radical hydroxyle ou un radical alkyle en C₁-C₁₈ linéaire ou ramifié ;
R₂ représente un radical ou un atome choisi parmi :
   - un atome d'hydrogène,
   - un radical alkyle en C₁ à C₁₈ linéaire ou ramifié,
   - un radical alcényle linéaire ou ramifiée en C₂ à C₁₈,
   - un radical perfluoroalkyle linéaire ou ramifié en C₁ à C₁₈,
   - un radical mono- ou polyhydroxyalkyle en C₁-C₈,
   - un radical cyclopentyle ou cyclohexyle,
   - un radical tétrahydropyranyle
   - un radical correspondant à la formule (II) suivante 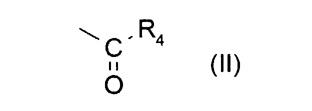 dans laquelle R₄ représente :
      - un radical alkyle en C₁-C₁₈ linéaire ou ramifié ;
      - un radical monohydroxyalkyle en C₁-C₈ ;
      - un radical polyhydroxyalkyle en C₃-C₆ renfermant de 2 à 5 groupes hydroxyles ;
      - un radical -NR'R" dans lequel R' et R" identiques ou différents représentent un atome d'hydrogène ou un radical alkyle en C₁-C₄ ou un radical alcényle en C₃-C₄ ou un radical cyclopentyle ou un radical cyclohexyle, R' et R" pouvant former un hétérocycle avec l'atome d'azote auquel ils se rattachent, le radical -NR'R" pouvant, en outre, être le reste d'un acide aminé ou de la glucosamine ;
      - un radical aralkyle ou un radical aryle éventuellement substitués par un ou plusieurs radicaux alkyles en C₁-C₁₈, linéaires ou ramifiés, ou un radical acyle -CO-R"' ou un radical alcoxy -OR"', dans lesquels R"' se définit comme précédemment ;
ainsi que les sels, les isomères et les stéréoisomères correspondants ;
étant entendu que si R₂ est un atome d'hydrogène, alors R₃ ne peut être un groupement N,N-diméthylaminoéthyle, ou un radical de formule : 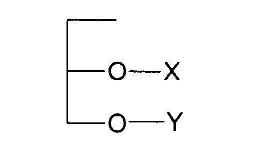 dans lequel X et Y identiques représentent un atome d'hydrogène ou un radical : 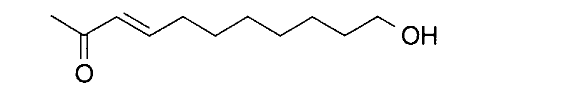 ou dans lequel X représente un atome d'hydrogène et Y représente un radical : 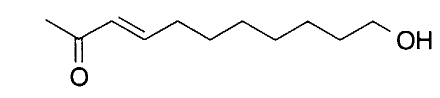 ou dans lequel X représente un radical : 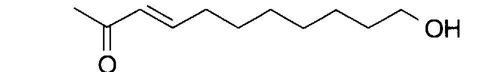 et Y représente : 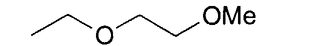 le composé ou la composition étant destiné à favoriser la desquamation de la peau et/ou à stimuler le renouvellement épidermique donc à lutter contre le vieillissement cutané.

Les sels des composés utilisables selon l'invention sont en particulier choisis parmi les sels de métal alcalin ou alcalino-terreux, ou encore parmi les sels de zinc, de magnésium ou de strontium, d'une amine organique ou les sels d'ammonium quaternaires, lorsqu'ils comportent au moins une fonction acide libre.

Les sels des composés conformes à l'invention sont en particulier choisis parmi les sels d'un acide minéral ou organique notamment les chlorhydrates, bromhydrates ou citrates, lorsqu'ils comportent au moins une fonction amine.

Selon une forme préférée de l'invention, on utilise un composé de formule (I) dans lequel R₁ est un groupement -NR'R" dans lequel R' est un atome d'hydrogène et R" représente un groupement mono ou polyhydroxyalkyle en C₁-C₆ .

Selon une autre forme préférée de l'invention, on utilise un composé de formule (I) dans lequel R₁ représente le radical -OR₃, dans lequel R₃ peut être un atome d'hydrogène, un groupement éthyle, un groupement carboxyméthyle, un groupement (carboxy-1')-éthyle ou un groupement (carboxy-1')-octyle, ou encore un groupement (carboxy-2')-phényle ou un groupement (carboxy-2' octanoyl-4')-phényle

Selon encore une autre forme préférée de l'invention, on utilise un composé de formule (I) dans lequel R₂ peut être un atome d'hydrogène ou un groupement hydroxy-2'-éthyl ou un groupement dihydroxy-2',3'-propyle ou un groupement hydroxy-2'-propyle ou un groupement -CO-R₄ dans lequel R₄ peut être un groupement hydroxyméthyle ou un groupement hydroxy-1'-éthyle, ou un groupement (hydroxy-1')-heptyle ou un groupement (hydroxy-2')-phényle ou un groupement (hydroxy-2' octanoyle-4')-phényle.

Les composés préférentiels utilisés selon l'invention sont ceux pour lesquels
- quand R₁ est le radical -OH alors R₂ peut être un atome d'hydrogène, un groupement hydroxy-2'-éthyle, un groupement dihydroxy-2',3'-propyle, ou un groupement hydroxy-2'-propyle ;
- quand R₂ est un atome d'hydrogène alors R₁ est un groupement -NR'R" dans lequel R' est un atome d'hydrogène et R" représente un groupement mono ou polyhydroxyalkyle C₁-C₆ ;
- quand R₂ est un atome d'hydrogène alors R₁ représente le radical -OR₃ dans lequel R₃ peut être un groupement carboxyméthyle, un groupement (carboxy-1')-éthyle ou un groupement (carboxy-1')-heptyle, ou encore un groupement (carboxy-2')-phényle ou un groupement (carboxy-2' octanoyl-4')-phényle ;
- quand R₂ est le groupement -CO-R₄, alors R₄ peut être un groupement hydroxyméthyle, un groupement hydroxy-1'-éthyle, un groupement (hydroxy-1')-heptyle, un groupement (hydroxy-2')-phényle, ou un groupement (hydroxy-2' octanoyle-4')-phényle.

Les composés de formule (I) utilisables selon l'invention peuvent être d'origine naturelle ou synthétique. Par origine naturelle, on entend un composé extrait de matériel naturel dans lequel il se trouve présent. Par origine synthétique on entend un composé préparé par synthèse chimique ou par biotechnologie.

Bien entendu, il est possible d'utiliser selon l'invention les composés de formule (I) seuls ou en mélange.

De même selon l'invention, les composés de l'invention peuvent être utilisés sous leur forme Cis ou Trans.

La quantité de composés de formule (I) utilisable selon l'invention dépend bien évidemment de l'effet recherché et doit être une quantité efficace pour favoriser la desquamation de la peau et/ou stimuler le renouvellement épidermique et donc lutter contre le vieillissement cutané intrinsèque et/ou extrinsèque.

A titre d'exemple la quantité de composé de formule (I) utilisable selon l'invention peut aller par exemple de 0,001% à 20% et de préférence de 0,01% à 5% du poids total de la composition.

Selon un autre aspect, l'invention a pour objet une composition pour favoriser la desquamation de la peau et/ou stimuler le renouvellement épidermique et donc lutter contre le vieillissement cutané intrinsèque et/ou extrinsèque, comprenant au moins un composé de formule (I).

Selon encore un autre aspect, l'invention a pour objet un procédé de traitement non thérapeutique pour favoriser la desquamation de la peau et/ou stimuler le renouvellement épidermique et donc lutter contre le vieillissement cutané intrinsèque et/ou extrinsèque, caractérisé en ce qu'on applique sur la peau une composition cosmétique comprenant un composé de formule (I).

L'invention a encore pour objet de nouveaux dérivés de l'acide 10-hydroxy-2-décénoique répondant à la formule générale (I'): 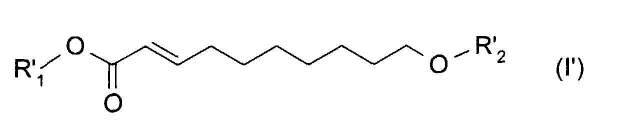 dans laquelle :
R'₁ représente un radical choisi parmi :
   - un radical H,
   - un radical de formule : 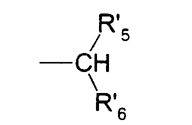
   - un radical de formule : 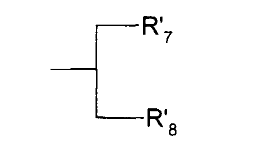
   - un radical de formule : 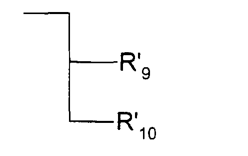 dans laquelle R'₅, R'₆, R'₇, R'₈, R'₉ et R'₁₀ ont les significations ci-après définies ;
R'₂ représente un radical ou un atome choisi parmi :
   - un atome d'hydrogène,
   - un radical de formule 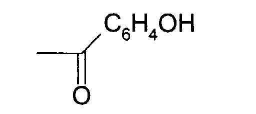
   - un radical de formule 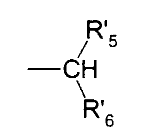
   - un radical de formule 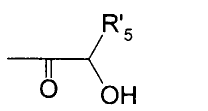
R'₅ pouvant représenter un atome d'hydrogène, un radical -CH₃, ou encore un radical -C₆H₁₃ ;
R'₆ pouvant représenter un radical -COOH, une radical -CO₂-CH₂-CH₃, ou encore un radical -CH₂OH ou un radical hydroxyle ;
R'₇ pouvant représenter un radical -OH, un radical de formule : 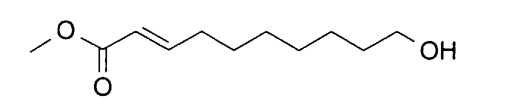 ou encore un radical de formule : 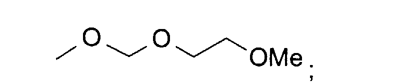
R'₈ pouvant représenter un radical -OH, ou encore un radical de formule : 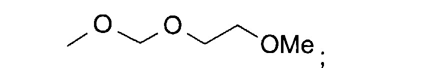
R'₉ pouvant représenter un radical de formule : 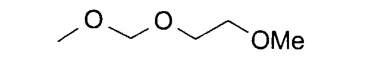
R'₁₀ pouvant représenter un radical de formule : 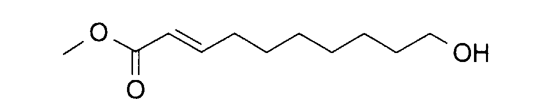 ou encore un radical de formule : 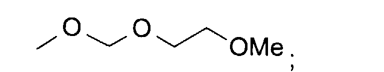 étant entendu que lorsque R'₁ est un radical -H alors R'₂ ne peut pas être un atome d'hydrogène,
   et que lorsque R'₇ est un radical de formule : 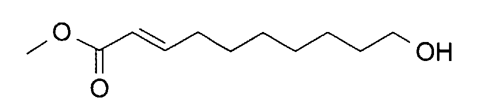 alors R'₈ ne peut pas être un radical de formule : 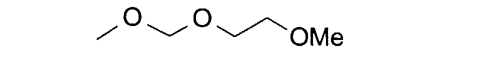 ainsi que les sels, les isomères et les stéréoisomères correspondants.

Les sels des composés conformes à l'invention sont en particulier choisis parmi les sels de métal alcalin ou alcalino-terreux, ou encore parmi les sels de zinc, de magnésium ou de strontium, d'une amine organique ou les sels d'ammonium quaternaires, lorsqu'ils comportent au moins une fonction acide libre. Les sels des composés conformes à l'invention sont en particulier choisis parmi les sels d'un acide minéral ou organique notamment les chlorhydrates, bromhydrates ou citrates, lorsqu'ils comportent au moins une fonction amine.

Un autre objet de l'invention concerne des compositions qui comprennent au moins un des composés répondant à la formule (I') définis ci-dessus.

Bien entendu, les compositions selon l'invention peuvent comprendre les composés de formule (I') seuls ou en mélanges en toutes proportions.

La quantité de composés de formule (I') contenue dans les compositions de l'invention est bien entendu fonction de l'effet recherché et peut donc varier dans une large mesure.

Pour donner un ordre de grandeur, la composition peut contenir au moins un composé de formule (I) en une quantité représentant de 0,001% à 20% du poids total de la composition et préférentiellement en une quantité représentant de 0,01% à 5% du poids total de la composition.

Les compositions de l'invention peuvent être ingérées, injectées ou appliquées sur la peau (sur toute zone cutanée du corps), les cheveux, les ongles ou les muqueuses (buccale, jugale, gingivale, génitale, conjonctive). Selon le mode d'administration, les compositions selon l'invention peuvent se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Les compositions selon l'invention peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition est une solution ou un gel huileux, la phase grasse peut représenter plus de 90% du poids total de la composition.

Les compositions de l'invention peuvent être à usage cosmétique ou pharmaceutique. Préférentiellement, les compositions de l'invention sont des compositions à usage cosmétique.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un composé de formule (I) à d'autres agents actifs. Parmi ces agents actifs, on peut citer à titre d'exemple :
- les agents améliorant la repousse et/ou le ralentissement de la chute des cheveux, et ayant déjà été décrits pour cette activité comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle, les dérivés de pyrimidine, comme le 2,4-diamino 6-piperidinopyrimidine 3-oxyde ou "Minoxidil" décrit dans les brevets US 4 139 619 et US 4 596 812, les agents favorisant la repousse des cheveux comme ceux décrits par la demanderesse dans la demande de brevet européen publiée sous le numéro 0648488 ;
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale, marine ou bactérienne.

A la liste ci-dessus, d'autres composés peuvent également être rajoutés, à savoir par exemple le Diazoxide, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés, des extraits d'origine végétale ou bactérienne.

Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale, marine ou bactérienne.

La composition pharmaceutique selon l'invention peut être administrée par voie parentérale, entérale ou encore par voie topique. De préférence, la composition pharmaceutique est administrée par voie topique.

Les composés de l'invention peuvent être synthétisés par des procédés tout à fait classiques généralement utilisés en synthèse organique.

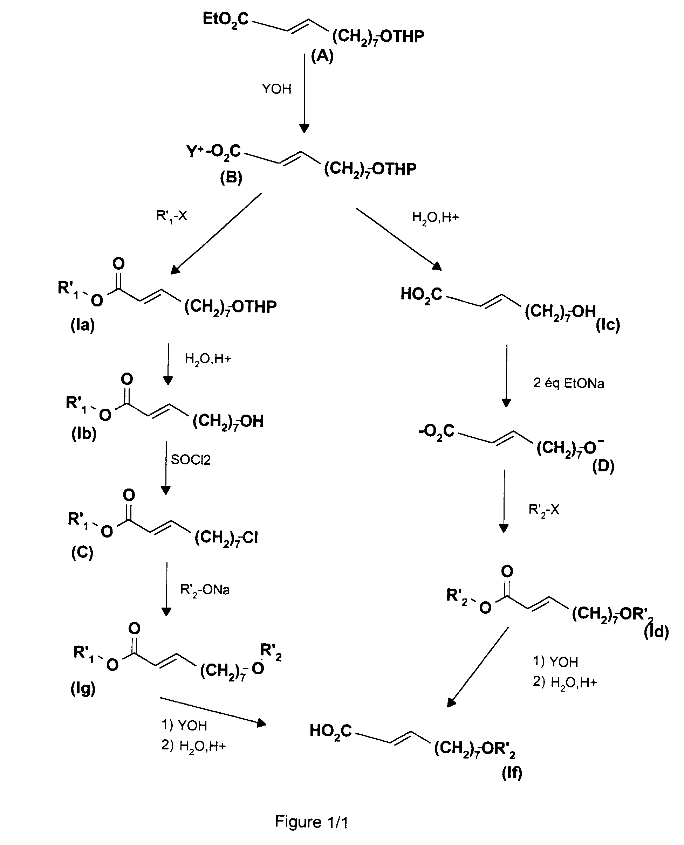

La figure (I) résume les voies de synthèse utilisables selon l'invention.

Les composés de l'invention sont obtenus à partir du 10-[(tétrahydro-2H-pyran-2-yl)oxy]-2-décènoate d'éthyle de formule (A, figure (I)) dont la préparation est décrite dans la littérature (MATSUI S., Bull. Chem. Soc. Jpn., 1984, 57 (2), 426-34 et VIG O., VIG A., MANN J., GUPTA K., J. Indian Chem. Soc., 1975, 52 (6), 538-40 par exemple).

Cet ester est transformé en sel de l'acide 10-[(tetrahydro-2H-pyran-2-yl)oxy]-2-décènoïque (formule B, figure (I)) par saponification à une température comprise entre 20°C et 60°C à l'aide d'un équivalent ou d'un excès (2 ou 3 équivalents) d'une base minérale telle que la soude ou la potasse (YOH, Y = Na ou K) agitée dans l'éthanol ou dans un solvant aprotique comme le diméthylformamide ou le diméthylsulfoxyde ou encore le tétrahydrofuranne. La transformation de l'ester éthylique en sel de structure (B) est suivie par chromatographie sur couche mince (CCM). A ce stade, selon le composé désiré, deux traitements du milieu réactionnel sont entrepris :
- pour obtenir les composés de formule générale (I') pour lesquels R'₁ est un radical -CHR'₅R'₆, on ajoute au mélange réactionnel contenant le sel de formule (B), un halogénure d'alkyle de structure R'₁-X, (R'₁=-CHR'₅R'₆, avec R'₅ et R'₆ tels que définis précédemment, X = halogène), les halogénures préférés étant les chlorures ou les bromures. L'anion carboxylate (-CO₂⁻) du dérivé de structure (B) déplace cet halogénure pour conduire aux esters de formule (la).
L'ester ainsi formé est alors isolé en versant ce mélange dans l'eau, puis en l'extrayant par un solvant organique tel qu'un solvant chloré comme le dichlorométhane ou le dichloro-1,2 éthane ou un solvant éthéré comme l'éther diéthylique. La phase organique est séparée, lavée à l'eau puis séchée sur sulfate de sodium et enfin concentrée.
L'ester brut (la) est à ce stade transformé en composé (Ib) par agitation dans l'eau acidulée (pH environ de 4) de façon à déprotéger la fonction alcool en position 10. Cette transformation est suivie par chromatographie sur couche mince (CCM) pour l'arrêter dès que la déprotection est réalisée sans que la fonction ester nouvellement formée soit modifiée. On peut également réaliser la déprotection de la fonction alcool en position 10 en traitant l'ester brut (la) par une résine acide comme par exemple une résine DOWEX 50W X2 préalablement activée, dans un solvant organique tel que le méthanol, à température ordinaire.
Le composé (Ib) obtenu est traité en présence d'au moins 1 équivalent d'un réactif tel que le chlorure de thionyle pour obtenir le chlorure correspondant (C). Ce chlorure est alors mis en réaction avec un alcoolate de structure R'₂-O⁻Na⁺ (R'₂ ayant les significations précédemment décrites), suivant les conditions habituelles de déplacement d'un halogénure par un alcoolate. On obtient ainsi les composés de formule générale (I') pour lesquels R'₁ est un radical -CHR'₅R'₆.
pour obtenir les composés de formule (I') pour lesquels R'₁ est un hydrogène, deux voies de synthèses sont possibles :
- à partir des composés de formule générale (I') pour lesquels R'₁ est un radical -CHR'₅R'₆ obtenus par la voie de synthèse précédemment décrite (Ig), par saponification à une température comprise entre 20°C et 60°C à l'aide d'un équivalent ou d'un excès (2 ou 3 équivalents) d'une base minérale telle que la soude ou la potasse (YOH, Y = Na ou K) agitée dans l'éthanol ou dans un solvant aprotique comme le diméthylformamide ou le diméthylsulfoxyde ou encore le tétrahydrofuranne puis agitation dans l'eau acidulée (pH environ de 4).
- au mélange réactionnel contenant le sel de l'acide 10-[(tetrahydro-2H-pyran-2-yl)oxy]-2-décènoïque (formule B, figure (I)), on ajoute une solution aqueuse d'acide chlorhydrique jusqu'à pH=1 sous agitation à température ambiante (20°C) jusqu'à transformation totale en acide 10-hydroxy 2-décènoïque de formule (Ic). Le mélange réactionnel est dilué par ajout de trois fois son volume d'eau, extrait par de éther diéthylique ou de l'acétate d'éthyle. Les phases organiques réunies sont lavées à l'eau, séchées sur sulfate de sodium et concentrées. L'acide brut est purifié ensuite par recristallisation dans un mélange acétate d'éthyle - éther de pétrole pour conduire à l'acide attendu sous forme de cristaux blancs.
   L'acide 10-hydroxy 2-décènoïque (Ic) est traité par deux équivalents d'éthylate de sodium dans l'éthanol. Par évaporation du solvant, on obtient le dianion de structure (D) que l'on fait réagir en milieu anhydre dans un solvant aprotique tel que le diméthylsulfoxyde ou le tétrahydrofuranne sur 2 équivalents d'un halogénure de structure R'₂-X (R'₂ ayant les significations précédemment décrites, X = halogène) pour obtenir les composés de structure générale (Id). Les composés pour lesquels R'₁=H peuvent être obtenus par saponification par 1 équivalent de base ou un léger excès Y-OH des composés (Id), puis acidification du milieu réactionnel. On obtient ainsi les composés de formule (I') pour lesquels R'₁ est un hydrogène.

Suivant le composé désiré de formule (I'), lorsque les synthèses nécessitent l'utilisation d'un halogénure de formule R'₁X ou R'₂X et que ces radicaux R'₁ ou R'₂ comportent des groupements -CO₂H, -CH₂OH ou -OH, il peut être nécessaire de protéger les hydroxyles avant mise en réaction des halogénures R'₁X ou R'₂X. Cette protection peut être par exemple assurée par un groupe triméthylsilyle et les groupements -CO₂H, -CH₂OH ou -OH sont alors transformés par réaction avec le chlorure de triméthylsilyle respectivement en ester-CO₂Si(CH₃)₃, en éther-CH₂-O-Si(CH₃)₃ ou -O-Si(CH₃)₃. Leur déprotection est alors effectuée en fin de réaction desdits halogénures R'₁X ou R'₂X par une hydrolyse en présence d'un catalyseur acide.

L'invention est illustrée plus en détail dans les exemples suivants.

Exemple 1: On étudie, dans cet exemple, la capacité de l'acide 10-hydroxy-2-décénoique à favoriser la desquamation.
Ce test de criblage *in vitro* d'un agent actif sur la desquamation est réalisé sur kératinocytes humains différenciés. Le principe du test repose sur le fait que la desquamation induit la libération de cornéocytes. Le pouvoir de desquamation du produit testé va être d'autant plus grand que le nombre de cornéocytes libéré sera important.

Le protocole du test a été le suivant: à partir de biopsies de peau humaine, les kératinocytes obtenus par séparation de l'épiderme sont dissociés par action enzymatique à la trypsine et mis en culture à la concentration de 2.10⁵ cellules/ml. La croissance et la différenciation des kératinocytes a été obtenue par culture durant 10 à 20 jours en milieu spécifique. Puis, après élimination du milieu de culture, l'activité du produit à tester est évalué. Pour ce faire, deux prélèvements à T0 et T60 ont été réalisés, c'est-à-dire avant l'ajout du produit et 60 minutes après cet ajout. Les prélèvements ainsi effectués ont été analysés au cytomètre de flux pour dénombrer la population de cornéocytes. Le cytomètre de flux permet de distinguer les populations de cornéocytes et de kératinocytes par traitement à l'acridine orange spécifique de l'ADN des cellules. Cette coloration est spécifique des kératinocytes puisque les cornéocytes normaux ne possèdent pas de noyaux donc pas d'ADN.

L'indice de détachement cellulaire est déterminé par la différence entre T60 et T0. La même mesure a été réalisée pour un témoin ne contenant pas de produit à tester car l'expérience produit inévitablement la libération de cornéocytes, même en absence d'actifs.

Le test a été effectué avec de l'acide 10-hydroxy-2-décénoique à la concentration de 5.10⁻⁵M.

Les résultats de cette étude sont résumés dans le tableau suivant :

| composés à 5 10⁻⁵ M | %** |
|---|---|
| Référence* | 92 |
| acide 10-hydroxy-2-décénoique (R₁ et R₂ = OH) | 110 |

| | |
|---|---|
| * Référence : Acide 2-hydroxy-5-octanoylbenzoïque connu comme favorisant la desquamation (Brevet FR-85-06953 de la demanderesse) | |
| ** : % d'activité par rapport au témoin constitué d'une culture identique en l'absence de composé. | |

Ces résultats montrent que l'activité de l'acide 10-hydroxy-2-décénoique sur le détachement cellulaire est importante.

Exemple 2: Exemples de formulations illustrant l'invention. Ces compositions ont été obtenues par simple mélange des différents composants.

### Composition 1 : Lait pour le visage

- Huile de vaseline 7,0 g
- Acide 10-hydroxy-2-décènoïque 1,0 g
- Monostéarate de glycéryle, stéarate de polyéthylène glycol (100 OE) 3,0 g
- Polymère carboxyvinylique 0,4 g
- Alcool stéarylique 0,7 g
- Protéines de soja 3,0 g
- NaOH 0,4 g
- Conservateur qs
- Eau qsp 100 g

Cette composition se présente sous forme d'un lait pour le visage, ayant de bonnes propriétés cosmétiques, et étant doux et confortable à utiliser. Le pH de la composition est d'environ 5,5.

### Composition 2 : Lotion

- 10-(dihydroxy-2',3' propyloxy) 2-décènoate d'éthyle 0,5 g
- Palmitate d'éthyl-2 hexyle 10,0 g
- Cyclopentadiméthylsiloxane 20,0 g
- Butylène glycol 5,0 g
- Conservateur qs
- Eau qsp 100 g

Cette lotion, qui ne contient pas de tensioactif, favorise la desquamation de la peau.

### Composition 3 : Lait

- Palmitate d'octyle 35,0 g
- Glycérine 2,0 g
- 10-(Ethoxycarbonyl-1' éthyloxy) 2-décènoate d'éthoxycarbonyl-1' éthyle 2,0 g
- Polymère réticulé acrylates C₁₀-C₃₀/alkylacrylates 0,1 g
- Triéthanolamine 0,1 g
- Acides aminés de blé 1,0 g
- Conservateur qs
- Eau qsp 100 g

Le lait obtenu, qui ne contient pas de tensioactif, possède de bonnes propriétés cosmétiques.

### Composition 4 : Gel pour le visage

- Glycérine 10,0 g
- 10-Hydroxy-2-décènoate d'éthoxycarbonyl-1' heptyle 2,0 g
- Cocoamphodiacétate de disodium 1,0 g
- Conservateur qs
- Eau qsp 100 g

Le gel obtenu possède de bonnes propriétés cosmétiques.

### Composition 5 : Gel nettoyant à l'eau

- Butylène glycol 7,0 g
- Sarcosinate de lauroyl sodium 4,0 g
- 10-(2'hydroxybenzoyloxy)-2-décènoate d'éthyle 5,0 g
- Triéthanolamine 0,8 g
- Carbomer 0,5 g
- Conservateur qs
- Eau qsp 100 g

Le gel obtenu possède de bonnes propriétés cosmétiques.

## Revendications

1. Utilisation en tant que composé actif dans une composition, d'une quantité efficace d'au moins un composé répondant à la formule générale (I): 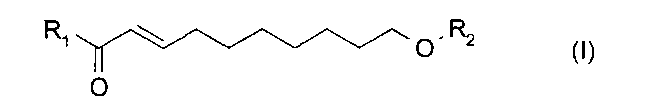 dans laquelle
R₁ représente un radical ou un atome choisi parmi :
- un atome d'hydrogène
- un radical -NR'R" dans lequel R' et R" identiques ou différents représentent un atome d'hydrogène ou un radical alkyle en C₁-C₄ ou un radical alcényle en C₃-C₄ ou un radical cyclopentyle ou un radical cyclohexyle, R' et R" pouvant former un hétérocycle avec l'atome d'azote auquel ils se rattachent, le radical -NR'R" pouvant, en outre, être le reste d'un acide aminé ou de la glucosamine.
- un radical -OR₃, dans lequel R₃ représente un radical ou un atome choisi parmi :
- un atome d'hydrogène ;
- un radical alkyle linéaire ou ramifiée en C₁ à C₁₈ ;
- un radical alcényle linéaire ou ramifiée en C₂ à C₁₈ ou
- un radical perfluoroalkyle linéaire ou ramifiée en C₁ à C₁₈ ;
les radicaux alkyle ou alcényle pouvant être substituées par un ou plusieurs groupements hydroxyles et/ou amino et/ou aminoalkyles ; ces radicaux peuvent en outre être substitués par une fonction acide carboxylique ou une fonction carboxylate d'alkyle linéaire ou ramifié en C₁-C₁₈ ;
- un radical aryle ou un radical aralkyle éventuellement substitués par des radicaux alkyles en C₁-C₁₈ linéaires ou ramifiés, des radicaux acyles -CO-R"', des radicaux alcoxy -OR"', dans lesquels R"' représente un atome d'hydrogène ou un radical hydroxyle ou un radical alkyle en C₁-C₁₈ linéaire ou ramifié.
R₂ représente un radical ou un atome choisi parmi :
- un atome d'hydrogène,
- un radical alkyle en C₁ à C₁₈ linéaire ou ramifié,
- un radical alcényle linéaire ou ramifiée en C₂ à C₁₈,
- un radical perfluoroalkyle linéaire ou ramifié en C₁ à C₁₈,
- un radical mono- ou polyhydroxyalkyle en C₁-C₈,
- un radical cyclopentyle ou cyclohexyle,
- un radical tétrahydropyranyle
- un radical correspondant à la formule (II) suivante 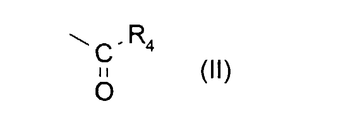 dans laquelle R₄ représente :
- un radical alkyle en C₁-C₁₈ linéaire ou ramifié ;
- un radical monohydroxyalkyle en C₁-C₈ ;
- un radical polyhydroxyalkyle en C₃-C₆ renfermant de 2 à 5 groupes hydroxyles ;
- un radical -NR'R" dans lequel R' et R" identiques ou différents représentent un atome d'hydrogène ou un radical alkyle en C₁-C₄ ou un radical alcényle en C₃-C₄ ou un radical cyclopentyle ou un radical cyclohexyle, R' et R" pouvant former un hétérocycle avec l'atome d'azote auquel ils se rattachent, le radical -NR'R" pouvant, en outre, être le reste d'un acide aminé ou de la glucosamine ;
- un radical aralkyle ou un radical aryle éventuellement substitués par un ou plusieurs radicaux alkyles en C₁-C₁₈, linéaires ou ramifiés, ou un radical acyle -CO-R"' ou un radical alcoxy -OR"', dans lesquels R"' se définit comme précédemment;
ainsi que les sels, les isomères et les stéréoisomères correspondants ;
étant entendu que si R₂ est un atome d'hydrogène, alors R₃ ne peut être un groupement N,N-diméthylaminoéthyle, ou un radical de formule : 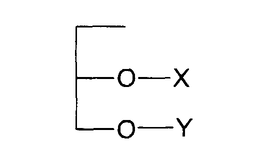 dans lequel X et Y identiques représentent un atome d'hydrogène ou un radical : 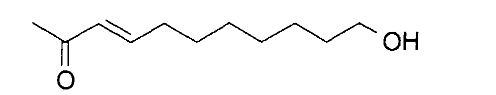 ou dans lequel X représente un atome d'hydrogène et Y représente un radical : 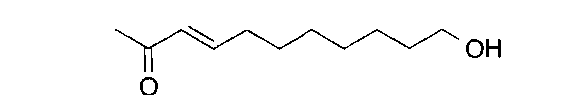 ou dans lequel X représente un radical : 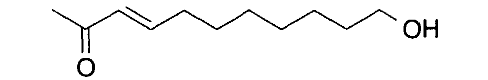 et Y représente : 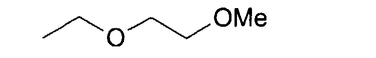 le composé ou la composition étant destiné à favoriser la desquamation de la peau et/ou à stimuler le renouvellement épidermique donc à lutter contre le vieillissement cutané.

2. Utilisation selon le revendication précédente, caractérisée par le fait que les sels des composés utilisables selon l'invention sont choisis parmi les sels de métal alcalin ou alcalino-terreux, ou encore parmi les sels de zinc, de magnésium ou de strontium, d'une amine organique ou les sels d'ammonium quaternaires.

3. Utilisation selon l'une quelconque des revendications précédentes, caractérisée par le fait que les sels sont choisis parmi les sels d'un acide minéral ou organique notamment les chlorhydrates, bromhydrates ou citrates, lorsqu'ils comportent au moins une fonction amine.

4. Utilisation selon l'une quelconque des revendications précédentes, caractérisée par le fait que le composé de formule (I) est un composé dans lequel R₁ représente le radical -OR₃, dans lequel R₃ peut être un atome d'hydrogène, un groupement carboxyméthyle, un groupement (carboxy-1')-éthyle ou un groupement (carboxy-1')-heptyle, ou encore un groupement (carboxy-2')-phényle ou un groupement (carboxy-2' octanoyl-4')-phényle.

5. Utilisation selon l'une quelconque des revendications précédentes, caractérisée par le fait que le composé de formule (I) est un composé dans lequel R₂ représente un atome d'hydrogène ou un groupement hydroxy-2'-éthyl ou un groupement dihydroxy-2',3'-propyle ou un groupement hydroxy-2'-propyle ou un groupement -CO-R₄ dans lequel R₄ peut être un groupement hydroxyméthyle ou un groupement hydroxy-1'-éthyle, ou un groupement (hydroxy-1')-heptyle ou un groupement (hydroxy-2')-phényle ou un groupement (hydroxy-2' octanoyle-4')-phényle.

6. Utilisation selon l'une quelconque des revendications précédentes, caractérisée par le fait que le composé de formule (I) est un composé dans lequel quand R₁ est le radical -OH alors R₂ peut être un atome d'hydrogène ou un groupement hydroxy-2'-éthyl ou un groupement dihydroxy-2',3'-propyle ou un groupement hydroxy-2'-propyle.

7. Utilisation selon l'une quelconque des revendications précédentes, caractérisée par le fait que le composé de formule (I) est un composé dans lequel quand R₂ est un atome d'hydrogène alors R₁ est un groupement -NR'R" dans lequel R' est un atome d'hydrogène et R" représente un groupement mono ou polyhydroxyalkyle en C₁-C₆.

8. Utilisation selon l'une quelconque des revendications précédentes, caractérisée par le fait que le composé de formule (I) est un composé dans lequel quand R₂ est un atome d'hydrogène alors R₁ représente le radical -OR₃ dans lequel R₃ peut être un groupement carboxyméthyle ou un groupement (carboxy-1')-éthyle ou un groupement (carboxy-1')-heptyle, ou encore un groupement (carboxy-2')-phényle ou un groupement (carboxy-2' octanoyl-4')-phényle.

9. Utilisation selon l'une quelconque des revendications précédentes, caractérisée par le fait que le composé de formule (I) est un composé dans lequel quand R₂ est le groupement -CO-R₄, alors R₄ peut être un groupement hydroxyméthyle ou un groupement hydroxy-1'-éthyle ou un groupement (hydroxy-1')-heptyle ou un groupement (hydroxy-2')-phényle ou un groupement (hydroxy-2' octanoyle-4')-phényle.

10. Utilisation selon l'une quelconque des revendications précédentes, caractérisée par le fait que le composé de formule (I) est utilisé en une quantité allant de 0,001% à 20% du poids total de la composition.

11. Utilisation selon l'une quelconque des revendications précédentes, caractérisée par le fait que le composé de formule (I) est utilisé en une quantité allant de 0,01% à 5% du poids total de la composition.

12. Composition pour favoriser la desquamation de la peau et/ou stimuler le renouvellement épidermique et donc lutter contre le vieillissement cutané intrinsèque et/ou extrinsèque, caractérisée en ce qu'elle comprend au moins un composé de formule (I) tel que défini dans l'une des revendications 1 à 10.

13. Procédé de traitement non thérapeutique pour favoriser la desquamation de la peau et/ou stimuler le renouvellement épidermique et donc lutter contre le vieillissement cutané intrinsèque et/ou extrinsèque, caractérisé en ce qu'on applique sur la peau une composition cosmétique telle que définie dans la revendication 12.

14. Nouveaux dérivés de l'acide 10-hydroxy-2-décénoique répondant à la formule générale (I'): 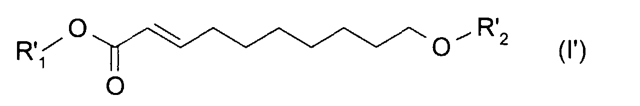 dans laquelle :
R'₁ représente un radical choisi parmi :
- un radical H,
- un radical de formule : 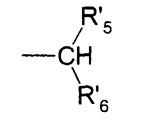
- un radical de formule : 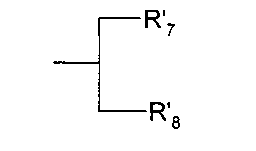
- un radical de formule : 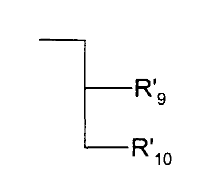 dans laquelle R'₅, R'₆, R'₇, R'₈, R'₉ et R'₁₀ ont les significations si après définies ;
R'₂ représente un radical ou un atome choisi parmi :
- un atome d'hydrogène,
- un radical de formule 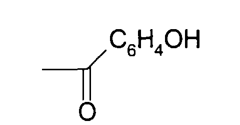
- un radical de formule 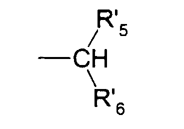
- un radical de formule 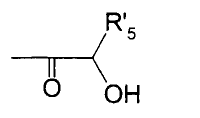
R'₅ pouvant représenter un atome d'hydrogène, un radical -CH₃, ou encore un radical -C₆H₁₃ ;
R'₆ pouvant représenter un radical -COOH, une radical -CO₂-CH₂-CH₃, ou encore un radical -CH₂OH ou un radical hydroxyle ;
R'₇ pouvant représenter un radical -OH, un radical de formule : 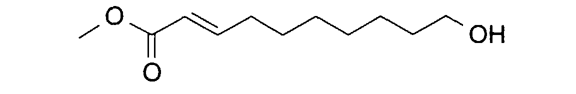 ou encore un radical de formule : 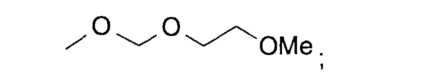
R'₈ pouvant représenter un radical -OH, ou encore un radical de formule : 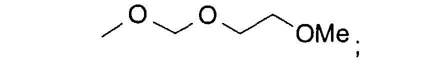
R'₉ pouvant représenter un radical de formule : 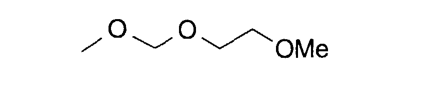
R'₁₀ pouvant représenter un radical de formule : 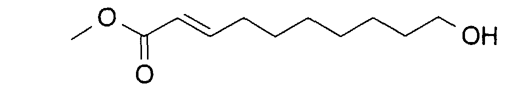 ou encore un radical de formule : 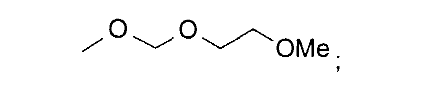 étant entendu que lorsque R'₁ est un radical -H alors R'₂ ne peut pas être un atome d'hydrogène,
et que lorsque R'₇ est un radical de formule : 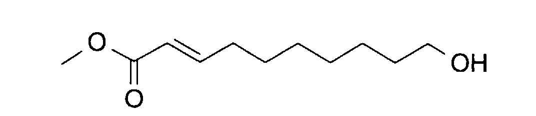 alors R'₈ ne peut pas être un radical de formule : 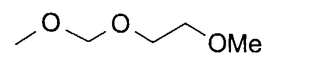 ainsi que les sels, les isomères et les stéréoisomères correspondants.

15. Composition comprenant au moins un composé de formule (I') tel que défini dans la revendication 14.

16. Composition selon la revendication 15, caractérisée par le fait que le composé de formule (I') en une quantité représentant de 0,001% à 20% du poids total de la composition.

17. Composition selon la revendication 16, caractérisée par le fait que le composé de formule (I') en une quantité représentant de 0,01% à 5% du poids total de la composition.
